# EUROPEAN PATENT APPLICATION

(11) **EP 2 679 264 A1**
(43) Date of publication of application: **01.01.2014**
(21) Application number: 12846543.2
(22) Date of filing: 01.11.2012
(51) Int. Cl.: A61M 11/02

(54) **GAS MIST INHALER**

(30) Priority: 04.11.2011 JP 2011242314
(71) Applicant: Nakamura, Shoichi, Higashichikuma-gun Nagano 399-7502 (JP); ACP Japan Co., Ltd., Tokyo 113-0033 (JP)
(72) Inventor: NAKAMURA, Shoichi, Higashichikuma-gun, Nagano 399-7502 (JP)
(74) Representative: Gill, David Alan
(86) International application number: PCT/JP2012/078310
(87) International publication number: WO 2013/065776

(57) **Abstract**

The present invention is to provide a gas mist inhaler, in which a gas introduction part of heightening pressure for supplying a gas mist is made integral with a gas mist generator in order to realize a simplified structure and cost reduction. This gas mist inhaler is provided with a gas supply means 10, a gas mist generation means 30 and an inhalation member 50, and the gas mist generation means 30 forms integrally a connection part to the gas supply means 10, a diverging part of branching a gas flow from the connection part, a liquid storage, a nozzle of discharging one-side gas flow branched by the diverging part, a liquid suction pipe of sending the liquid to a front end of the nozzle, a collision member of colliding the liquid blown up by the gas flow discharged from the nozzle with this collision member, a confluent part of joining the generated gas mist with the gas from an upward side, a gas introduction part of leading the other-side gas flow branched by the diverging part until the confluent part, and a gas mist discharge part of collecting and discharging the generated gas mist and gas.

## Description

### TECHNICAL FIELD

The present invention relates to a gas mist inhaler for performing inhalation of a gas mist into a living body, in which the gas mist is prepared by pulverizing a liquid into micron sized mists and dissolving oxygen, carbon dioxide, or a mixed gas of oxygen and carbon dioxide.

### BACKGROUND OF THE INVENTION

Conventionally, it has been known that carbon dioxide (carbonic acid anhydride: CO₂) has two properties of being not only soluble in water (water-soluble) but also soluble in fat (fat-soluble) and, owing to having such both properties, when only contacting to the skin and the mucous membrane of a living body which are like as mixed with water and fat, carbon dioxide penetrates under a subcutaneous tissue and expands blood vessels around the penetrated parts, and it works to improve the blood circulation. By this action of accelerating the blood circulation, it displays various physiological effects such as dropping of blood pressure, improving of metabolism or accelerating to remove pain substances or waste products. Further, it has also anti-inflammation and anti-bacterial function. Therefore, carbon dioxide has recently been given attentions also from viewpoints of improving health or accelerating beauty other than the purpose of medical cares.

In the tissue of the living body, carbon dioxide works to release oxygen having been carried in combination with hemoglobin existing in a red blood cell. Around parts at the high concentration of carbon dioxide, the red blood cell releases more oxygen. Thus, supply of oxygen to cells by the red blood cell is mainly controlled by carbon dioxide. In short, being without carbon dioxide, hemoglobin remains as having been combined with oxygen and the cell becomes unable to receive oxygen. Carbon dioxide seems to be waste products resulted from action of the cell, however, as is seen, it plays in fact very important roles in the living body.

Further, recently, oxygen of the high concentration has also widely been known as effective over activity of metabolism, acceleration of blood circulation, fatigue recovery, or stability of blood pressure. Other than them, oxygen has effects of disinfection or sterilization by oxidation.

By the way, for easing a symptom of disease in a respiratory system such as asthma or allergic rhinitis, oral nasogastric inhalation of medicines or steam using an inhaler or a nose spray have been till now carried out.

An inventor of this invention has invented a gas mist inhaler, in which carbon dioxide or oxygen is efficiently dissolved in a liquid to turn out a mist for causing a physiological action of these gases to effectively influence to the living body. For example, the gas supplied into the gas mist generator is divided into two branches for using one part to generate the gas mist, while for supplying the other toward a discharge part from a gas mist generation part in order to heighten gas mist supply pressure of the gas mist generator.

### SUMMARY OF THE INVENTION

### PROBLEMS THAT THE INVENTION IS TO SOLVE

However, in the prior art gas mist inhaler, gas was branched within the gas mist generator, and guided to a gas mist generation part the through such as an outside installed tube. A case of using as above the outside installed tube was involved with problems of taking labors and at the same time increasing production costs. Further, if gas pressure is high, gas probably leaks from a connecting part of a tube.

Then, in view of the above mentioned circumstances, the invention is to provide such a gas mist inhaler, in which a gas introduction part for heightening pressure to supply the gas mist is made integral with a gas mist generator in order to realize a simplified structure and cost reduction.

### MEANS FOR SOLVING THE PROBLEMS

For solving the above mentioned problems, the invention is to provide such a gas mist inhaler, which comprises a gas supply means of supplying oxygen, carbon dioxide, or mixed gas (called as "gas" hereafter) of oxygen and carbon dioxide; a gas mist generation means of generating a mist (called as "gas mist" hereafter) prepared by pulverizing and dissolving the gas supplied from the gas supply means as well as the liquid stored in an inside thereof, and of supplying the mist under a condition of mixing with the gas; and an inhalation member connected to the gas mist generation means and having an inhalation port for causing the above mentioned gas mist to be absorbed into the living body; wherein the above mentioned gas mist generation means is integrally provided with a connection part to the gas supply means; a diverging part of branching a gas flow from the connection part; a liquid storage of storing the liquid; a nozzle of discharging one-side gas flow branched by the diverging part; a liquid suction pipe of sending the liquid to a front end of the nozzle; a collision member of colliding the liquid blown up by the gas flow discharged from the nozzle with this collision member; a confluent part of joining the generated gas mist with the gas from an upward side; a gas introduction part of leading the other-side gas flow branched by the diverging part until the confluent part; and a gas mist discharge part of collecting and discharging the generated gas mist and gas.

By the way, the invention refers it as "pulverizing and dissolving" to pulverize the liquid into fine liquid drops, and cause to contact and mix with gas (oxygen or carbon dioxide, or the mixed gas of oxygen and carbon dioxide).

Herein, with respect to the gas mist inhaler of this invention, in the gas mist generation means, it is desirable that the at least liquid storage is made replaceable and exchangeable with another liquid storage.

Herein, the gas mist generating means has preferably an air hole for taking in outside air, otherwise the inhalation member to have also sufficiently a further opening for taking in outside air.

The gas supply means is desirably a gas bomb of a cartridge system. Further, the gas supply means may have any one or a plurality of a gas supply time setting part, a gas supply pressure adjusting part and a gas mixing ratio setting part.

In addition, the gas mist generating means also may supply the gas mist into the plural inhalation members.

Next, it is best that the above mentioned liquid is any one or plural combination of water, ionic water, ozone water, physiological salt solution, purified water, or sterilized and purified water.

A size of the mist supplied from the gas mist generating means into the inhalation member is suitably not larger than 10 µm.

Desirably, the gas mist generating means has a gas mist supply pipe for supplying the gas mist and gas into the inhalation member, and this gas mist supply pipe is preferably furnished with a filter for removing liquid drops attached to the inside of the pipe. Still further, the gas mist supply pipe is suitably composed of a cornice shaped pipe over a whole or at one part of the gas mist supply pipe. The cornice shaped pipe is suitably formed inside with a groove in an axial direction of the pipe. In addition, this gas mist supply pipe is provided with a check valve.

Preferably, the gas mist generating means is in advance sterilized.

### EFFECTS OF THE INVENTION

According to the gas mist inhaler of the invention, by making the gas introduction part for heightening pressure of supplying the gas mist integrate with the gas mist generator, the structure of the invention is simplified to curtail using labors, enabling to reduce production cost and avoid gas leakage.

### BRIEF DESCRIPTION OF THE DRAWINGS

[FIG. 1] A generally schematic view of the gas mist inhaler depending on the first embodiment of the invention;
[FIG. 2] A typical view in cross section showing the structure of the gas mist generator in the gas mist inhaler of FIG. 1;
[FIG. 3] A perspective view in cross section of the gas mist generator seeing from an arrow A direction of FIG. 2;
[FIG. 4] A perspective view in cross section of the gas mist generator seeing from an arrow B direction of FIG. 2;
[FIG. 5] A partially enlarged cross sectional view of the gas mist generator in the gas mist inhaler in FIG. 1;
[FIG. 6] Typical views showing examples of the inhalation members used to the gas mist inhaler of the invention;
[FIG. 7] A typical view showing the example of the gas mist supply pipe connecting the gas mist generator to the inhalation member of the invention;
[FIG. 8] A cross sectional and perspective view of the gas mist supply pipe shown in FIG. 7;
[FIG. 9] A typical view showing a general appearance when housing the gas supply means and the gas mist generator in a case;
[FIG. 10] Typical views showing examples of using the cases showing in FIG. 9;
[FIG. 11] A generally schematic view of the gas mist inhaler depending on a second embodiment of the invention; and
[FIG. 12] A generally schematic view of the gas mist inhaler depending on a third embodiment of the invention.

### EMBODIMENTS FOR PRACTISING THE INVENTION

In the following description, explanations will be made to embodiments of this invention, referring to the attached drawings.

### [First Embodiment]

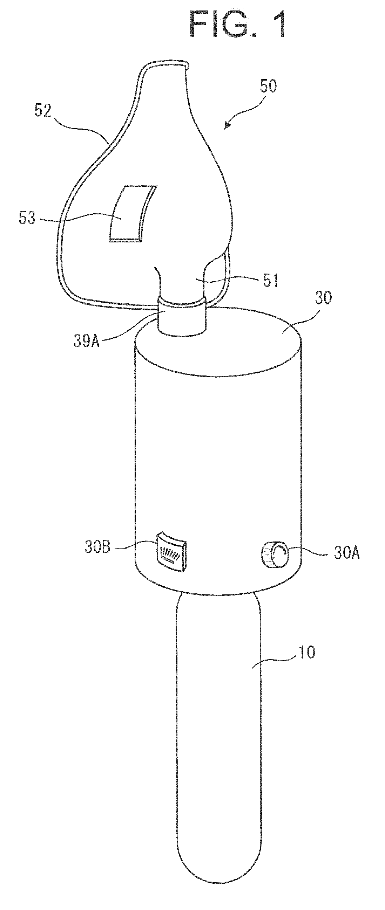

FIG. 1 is the generally schematic view of the gas mist inhaler in dependence on the first embodiment of this invention. As shown in FIG. 1, the gas mist inhaler of the present embodiment comprises a gas supply means 10 for supplying oxygen, carbon dioxide, or the mixed gas (called briefly as "gas" hereafter) of oxygen and carbon dioxide, a gas mist generator 30 being a gas mist generating means, and an inhalation mask 50 serving as an inhalation member of causing the generated gas mist to be absorbed into a living body.

The gas supply means 10 supplies gas to a connection part 31 of a later mentioned gas mist generator 30. In the present embodiment, as shown in FIG. 1, a small sized cartridge system is used paying attention to portability, and a cassette gas bomb or a gas container of high pressure for official uses are also sufficient.

The gas mist generator 30 previously stores inside the liquid, generates the gas mist by pulverizing and dissolving the liquid and gas by high speed flowing of the gas supplied from the gas supply means 10, further mixes the gas, and supplies the gas mist into the inhalation mask 50.

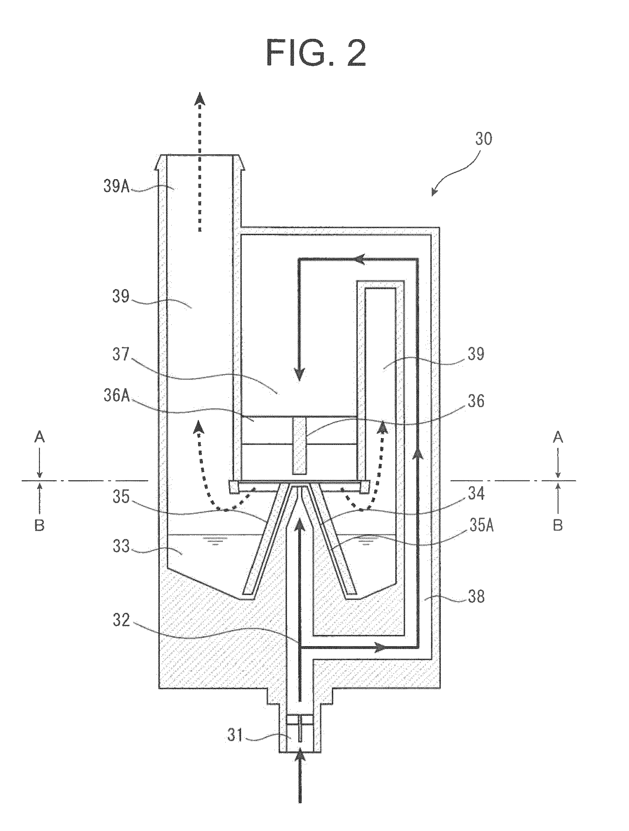

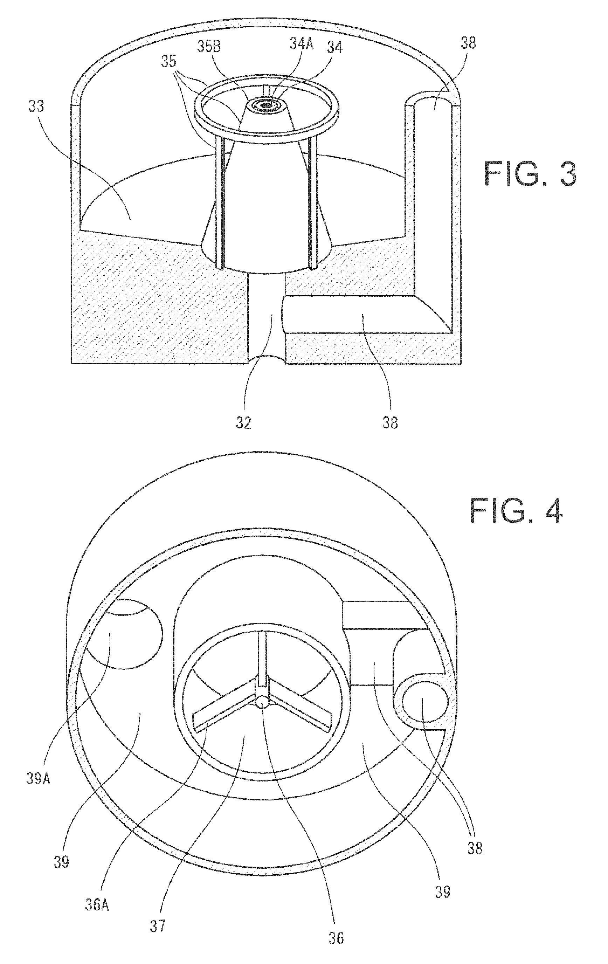

FIG. 2 is the typical view in cross sectional showing the structure of the gas mist generator 30. FIG. 3 is the perspective view in cross section of the gas mist generator 30 seeing from an arrow A direction of Fig. 2. By the way, FIG. 3 omits a wall part and the connection part 31 in the front side of a liquid storage 33. Fig. 4 is the perspective view in cross section seeing from the arrow B direction of FIG. 2.

As showing in these Figures, the gas mist generator 30 has a connection part 31 connected with a gas supply means 10, a diverging part 32 of branching the gas flowing from the connection part 31, a liquid storage 33 of storing the liquid, a nozzle 34 of discharging one-side gas flow branched by the diverging part 32, a liquid suction pipe 35A of sending the liquid to the front end of the nozzle 34, a baffle (collision member) 36 of colliding the liquid blown up by the gas flow discharged from the nozzle with this baffle, a confluent part 37 of joining the generated gas mist with the gas from an upward side, a gas introduction part 38 of leading the other-side gas flow branched by the diverging part 32 until the confluent part 37, and a gas mist discharge part 39 of collecting and discharging the generated gas mist, and those members are integrally formed.

To the connection part 31, the gas supply means 10 is, as illustrated in FIG. 1, connected directly, otherwise, via a gas code. The connection part 31 has such as a structure enabling to connect, by one touch, the gas supply means 10 or a gas code connected to the gas supply means 10, and in accordance with the gas supply means 10 to be connected, various forms may be employed. In a case of directly connecting the gas bomb as FIG. 1, the connection part 31 may have inside a regulator for adjusting a gas flowing amount from the gas supply means 10. FIG. 1 shows an example of furnishing a dial switch 30A and a residual gage 30B around the connection part 31. The dial switch 30A turns to enable ON-OFF of a gas supply and to adjust a gas supply amount. The residual gage 30B shows a gas residual amount of the gas supply means 10.

The gas supplied from the gas supply means 10 via the connection part 31 is branched into two routes at the diverging part 32. One of them directs to the nozzle 34, while the other to the gas introduction part 38. The gas having directed to the nozzle 34 is discharged out of a front end open 34A of the nozzle. On the other hand, the gas to the gas introduction part 38 is guided to the confluent part 37.

The liquid storage 33 has been stored and sealed with a predetermined liquid in advance when having built at a stage of setting up the system of this invention. When using, this liquid storage is opened to perform inhalation of the gas mist. Herein, as the liquid stored in the liquid storage 33, it is preferable to employ water, ionic water, ozone water physiological salt solution, purified water or sterilized and purified water. Further, these liquids are sufficient to contain medicines useful to users' diseases or symptom. As the medicines, for example, listed are anti-allergic agent, anti-inflammatory agent, anti-febrile agent, anti-fungus agent, anti-influenza virus agent, influenza vaccine, steroid agent, anti-cancer agent, or anti-hypertensive agent.

At the bottom center of the liquid storage 33, the nozzle 34 is placed. This nozzle 34 protrudes from the bottom of the liquid storage 33, and is squeezed in size toward the baffle 36 to be formed almost as a circular cone. The nozzle 34 is connected, at its base end, to one side of the diverging part 32, and the nozzle 34 enables to discharge gas from its front end open 34A.

The liquid suction pipe 35A is formed between the outer circumference of the nozzle 34 and a liquid suction pipe-forming member 35 of the almost circular cone being larger by one turn than the nozzle 34. That is, as shown in FIG. 2, by positioning as covering the liquid suction pipe-forming member 35 over the nozzle 34, a liquid suction pipe 35A is defined between the outer circumference of the nozzle 34 and the inner circumference of the liquid suction pipe-forming member 35. Although having omitted to show, since a minute nail shaped projection is provided at a base end (the lower part of the almost circular cone part) of the liquid suction pipe-forming member 35, a space is defined on the bottom between a base of the liquid suction pipe-forming member 35 and the bottom of the liquid storage 33, and from this space the liquid stored in the liquid storage 33 is sucked up by the liquid suction pipe 35A. In addition, the front end 35B of the liquid suction pipe-forming member 35 opens nearly the front end open 34A of the nozzle 34, and the liquid sucked up by the liquid suction pipe 35A collides with the gas flow discharged from the nozzle 34.

The liquid sucked up by the liquid suction pipe 35A collides with the gas flow discharged from the nozzle 34 and is blown up, struck against the baffle 36 placed in opposition to the front end open 34A of the nozzle 34 and crashed to turn out the gas mist. Herein, the baffle 36 is fixed to the inside wall of the confluent part 37 by a baffle supporter 3 6A, but may be fixed to a liquid suction pipe-forming member 35.

On the other hand, the gas branched at the diverging part 32 into the gas introduction part 38 gets to the confluent part 37 following the gas introduction part 38. The gas introduction part 38 is such a guiding path which passes the side face of the inside of the gas mist generator 30 from the diverging part 32 equipped at the lower part of the gas mist generator 30 and goes toward an upper part, and this gas introduction part 38 is formed integrally in the gas mist generator 30. The confluent part 37 is made of a cylindrical member disposed as encircling the baffle 36 above the front end open 34A of the nozzle 34, and communicates with the gas introduction part 38. Accordingly, the gas branched at the diverging part 32 and guided to the gas introduction part 38 confluents with the gas mist generated in the confluent part 37 at the upper part, and pushes out the gas mist toward a gas mist discharge part 39 formed around the cylindrical confluent part 37.

The gas supplied from the gas introduction part 38 into the confluent part 37 can be controlled in supply pressure owing to the size of a diameter of the gas introduction part 38. By controlling gas supply pressure, the gas mist supplying amount of the gas mist generator 30 is also can be controlled. Further, concentration of the gas mist (mist concentration in gas) or grain sizes of the mist can be also controlled by the diameter size in the gas introduction part 38.

A gas mist discharge part 39 is a space formed around the cylindrical confluent part 37, collects a gas mist driven out from the confluent part 37 by gas from the gas introduction part 38, and discharges the gas mist together with the gas. The gas mist driven out into the gas mist discharge part 39 is discharged into an inhalation mask 50 from the gas mist discharge mouth 39A opening at the upper part of the gas mist generator 30. As later mentioned, an interval between the gas mist discharge mouth 39A and the inhalation mask 50 may be connected by a gas mist supply pipe 43.

The gas mist generator 30 may be made such a structure which displaces a part including the at least liquid storage 33 and replaces another new liquid storage 33. That is, the gas mist generator 30 is made an assembling system and a replacing part including the liquid storage 33 is set up with another part, thereby enabling to accomplish a gas mist generator 30 integrating with the gas introduction part 38. Thus, if making the liquid storage 33 replaceable, the liquid storage 33 may be disposable enabling to keep hygiene. By making the liquid storage 33 replaceable, it is possible to omit a structure of supplementing the liquid into a liquid suction pipe 35A and to realize a device to be compact.

Any of the confluent part 37 or the gas introduction part 38, otherwise, the gas mist discharge part 39 may be provided with an air hole for taking in outside air. The gas mist generator 30 is desirably treated with sterilization when having built at a stage of setting up the system of this invention.

The inhalation mask 50 is such a member having a shape covering a user's respiratory organs (herein, the nose and mouth) for the user to easily breath the generated gas mist. The inhalation mask 50 is connected to the gas mist discharge mouth 39A via the connection part 51, and the user breathes the gas mist from the inhalation port 52. By the way, the inhalation mask 50 is preferably furnished with an open 53 for taking in outside air.

In the following, explanation will be made to one example of a sequence of inhaling the gas mist with the gas mist inhaler of the above mentioned first embodiment.

At first, the sealed gas mist generator 30 is opened to connected to the gas supply means 10 and the dial switch 30A is turned ON. Incidentally, this dial switch 30A can adjust a gas flowing amount.

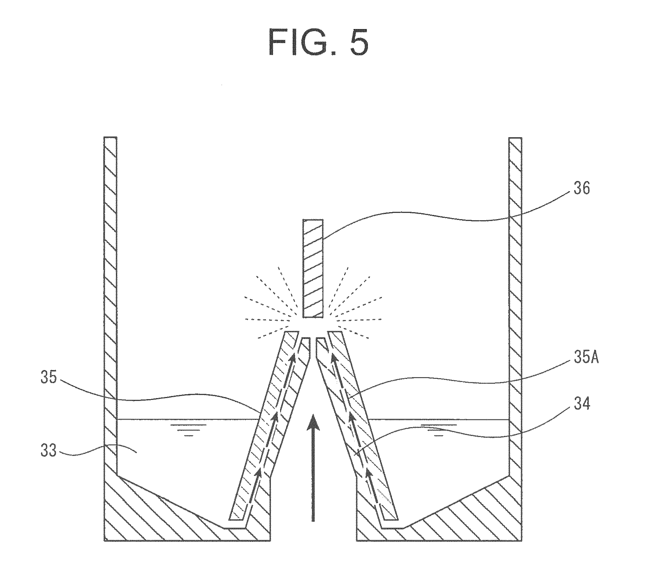

When gas is supplied into the nozzle 34, since the nozzle 34 is reduced in size toward the front end as shown in FIG. 5, the gas increases the flowing speed and is exhausted. The liquid is sucked up within the liquid suction pipe 35A owing to negative pressure caused by air flow of this time, is blown up by gas at the front end part 35B of the liquid suction pump 35A, and collides against the baffle 36, so that the mist is generated. Desirably, the diameter of the mist generated by this collision is fine, and concretely, best is not larger than 10 µm. The thus finely pulverized mist can display effects of minus ion.

The gas further passes through the diverging part 32 and is guided to the confluent part 37 from the gas introduction part 38, and heightens the exhausting pressure of the generated gas mist. The generated gas mist is mixed with the gas from the diverging part 32 and exhausted from the gas mist discharge mouth 39A into an inhalation mask 50. The gas mist and gas exhausted in the inhalation mask 50 can be inhaled from an inhalation port 52.

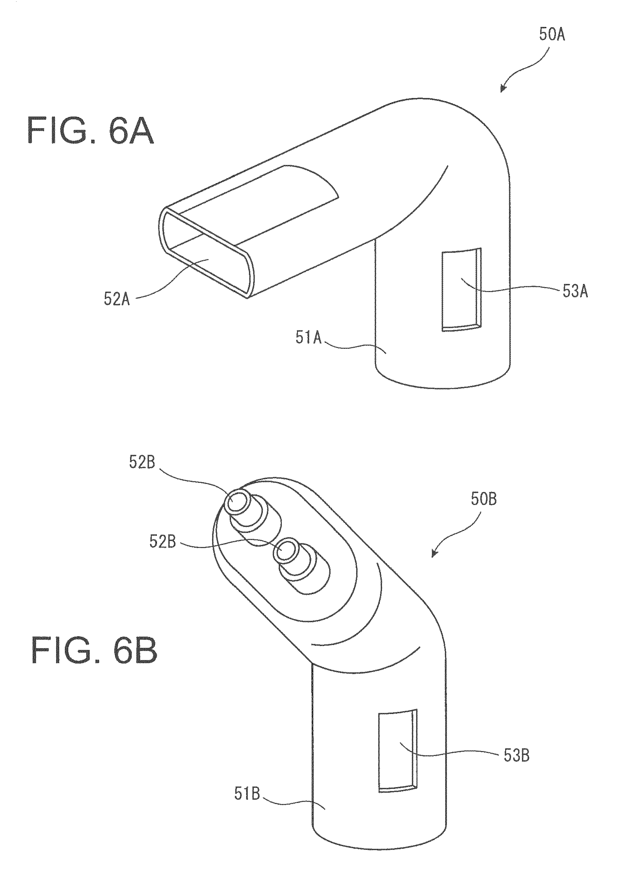

The above reference has showed, as the inhalation member, an example of using the inhalation mask 50 of a type covering the nose and the mouth, and other various types using the inhalation members are also available. FIGs. 6A and 6B show other examples of the inhalation members.

FIG. 6A is a mouth mask 50A of a mouth piece type used for inhaling from the mouth only. At the connection part 51A, this is connected to the gas mist discharge mouth 39A, and the user inhales the gas mist and gas into the mouth from the inhalation port 52A. The mouth mask 50A is suitable to have an opening 53A for taking in outer air. This mouth mask 50A is especially suited to moderate and cure symptoms of a throat.

FIG. 6B is a nose mask 50B of a nose piece type used for inhaling from the nose only. At the connection part 51B, this is connected to the gas mist discharge mouth 39B, and the user inhales the gas mist and the gas into the nose from the inhalation port 52B. The nose mask 50B is suitable to have an opening 53B for taking in outer air. This nose mask 50B is especially suited to moderate and cure allergic rhinitis of the nose.

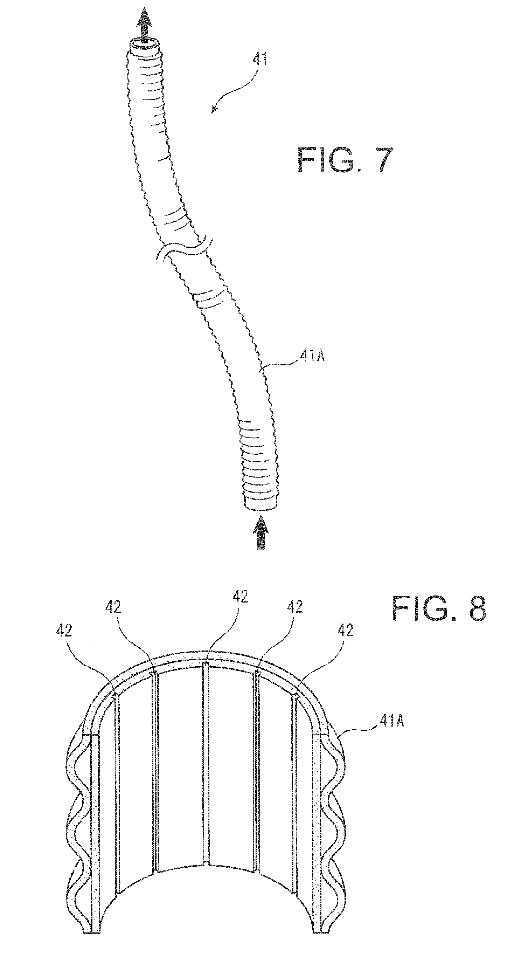

The above references have shown the examples of directly connecting the gas mist discharge mouth 39A and the connection part 51 of the inhalation mask 50, and the interval between the gas mist discharge mouth 39A and the inhalation mask 50 may be also connected via a pipe (gas mist supply pipe) as a tube. FIGs. 7 and 8 show an outline of the gas mist supply pipe.

The gas mist supply pipe 41 is desirably composed wholly or partially with a soft and cornice shaped pipe 41A of large diameter as shown in FIG. 7, and it is freely bent or expanded and contracted so that the user's action is not limited. In addition, as shown in FIG. 8, the cornice shaped pipe 41A is formed inside with grooves 42 in an axial direction of the pipe. By thus forming inside the grooves 42 of the pipe 41A, if the gas mist flowing in the gas mist supply pipe 41 becomes liquefied, it is easy to collect and recover liquid drops. FIG. 8 illustrates the inside of the cornice shaped pipe 41A is smooth, and may be formed to be cornice shape as the outside. Also in such a case, by forming the grooves 42, the liquefied gas mist is recovered.

The gas mist supply pipe 41 is provided inside with a check valve to avoid back flows of the gas mist and gas. Further, though not showing, the gas mist supply pipe 41 is preferably provided with a droplet removing filter to remove extra droplets attaching to the inside of the pipe.

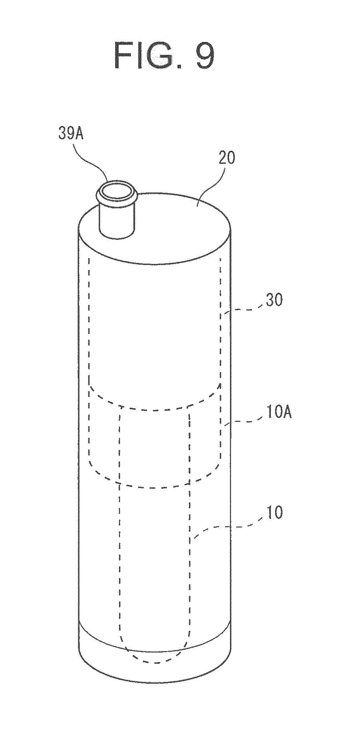

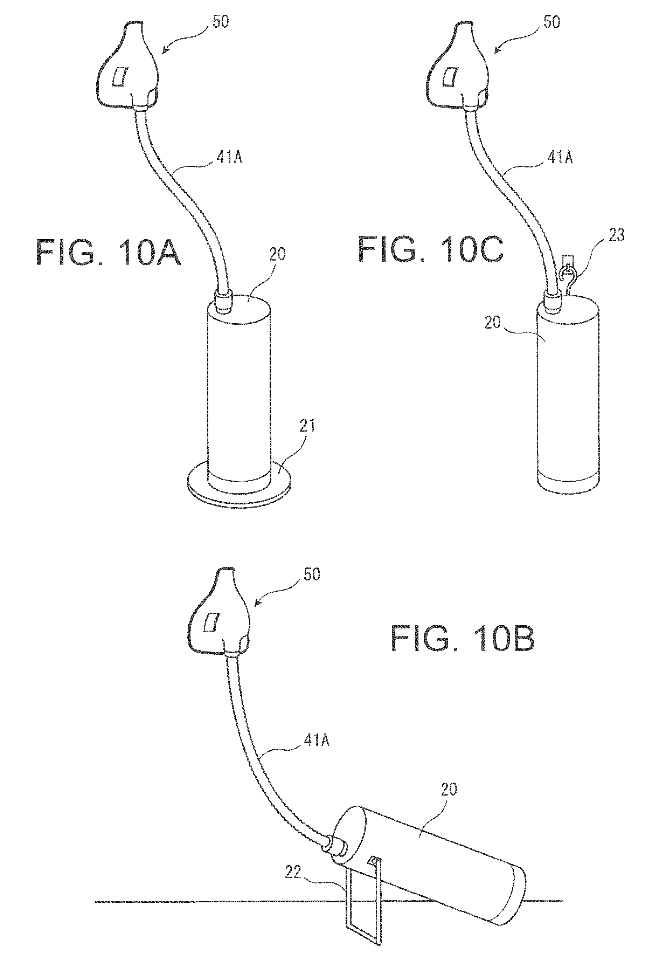

If a simple means like the cartridge system gas bomb as shown in FIG. 1 is used for the gas supply means 10, it is possible to use the gas supply means 10 and the gas mist generator 30 under a compact condition of containing them in the case 20 as showing in FIG. 9. Herein, the gas supply means 10 has a regulator. In FIG. 10, examples of using conditions are shown. As showing in FIGs. 10A to 10C, the cases 20 have stands 21, 22 or a hook 23 to stand upright, or the case 20 is suspended from a wall, so that the gas mist generator 30 is used as standing as possible. By such manners, the liquefied gas mist is easily recovered.

### [Second Embodiment]

The above first embodiment has shown the example as the gas supply means 10 using the small sized gas bomb of the cartridge system, and the present embodiment will show an example using a gas supply device of a stationary type.

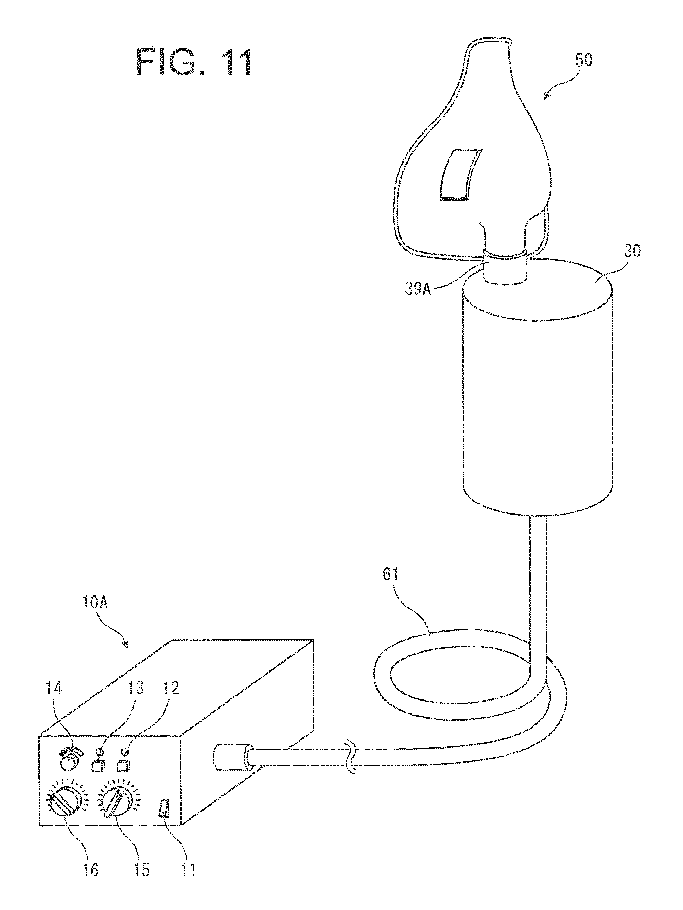

FIG. 11 is the generally schematic view of the gas mist inhaler depending on the second embodiment of this invention. As to the same parts as those of the embodiment shown in FIGs. 1 to 10, the same numerals will be given, and detailed explanation will be omitted.

As showing in FIG. 11, the gas mist inhaler of this embodiment has the gas supply device 10A as the gas supply means, the gas mist generator 30 as the gas mist generating means and the inhalation mask 50 as the inhalation member.

The gas supply device 10A is a stationary typed device for supplying gas into the gas mist generator 30 at predetermined pressure. Its inside is built-in with the gas bomb (not shown), and may be built-in with a compressor, otherwise may be connected to an external gas bomb.

The gas supply device 10A is, for example as shown in FIG. 11, provided with a power switch 11, oxygen supply ON/OFF switch 12, carbon dioxide supply ON/OFF switch 13, gas mixing ratio setting part 14, OFF timer (gas supply time setting part) 15, and gas supply pressure adjusting part 16. That is, in the present embodiment, the gas mist generation means 30 has neither a dial switch nor a residual gage, but the gas supply device 10A performs various controls. The gas supply device 10A and the gas mist generator 30 are connected via a gas supply pipe 61.

The gas mixing ratio setting part 14 is so composed as to regulate voluntarily mixing ratios of carbon dioxide and oxygen. It is thereby possible to reply at will to the user' s requests, for example, for lightening asthma or recovering fatigue such as setting the mixing ratio of oxygen to be much, or for accelerating a blood circulation such as setting the mixing ratio of carbon dioxide to be much. Further, the OFF timer 15 is for setting the gas supplying time, and when a set time passes away, the gas supplying is automatically stopped. The gas supply pressure adjusting part 16 can set the gas supplying pressure arbitrarily. Since the gas supplying time or pressure are possible thereby, its using scope can be broadened.

### [Third Embodiment]

The above first and second embodiments have shown the example where one gas mist generator 30 is connected with one inhalation member 50, and the present embodiment will show a structure of connecting a plurality of inhalation members.

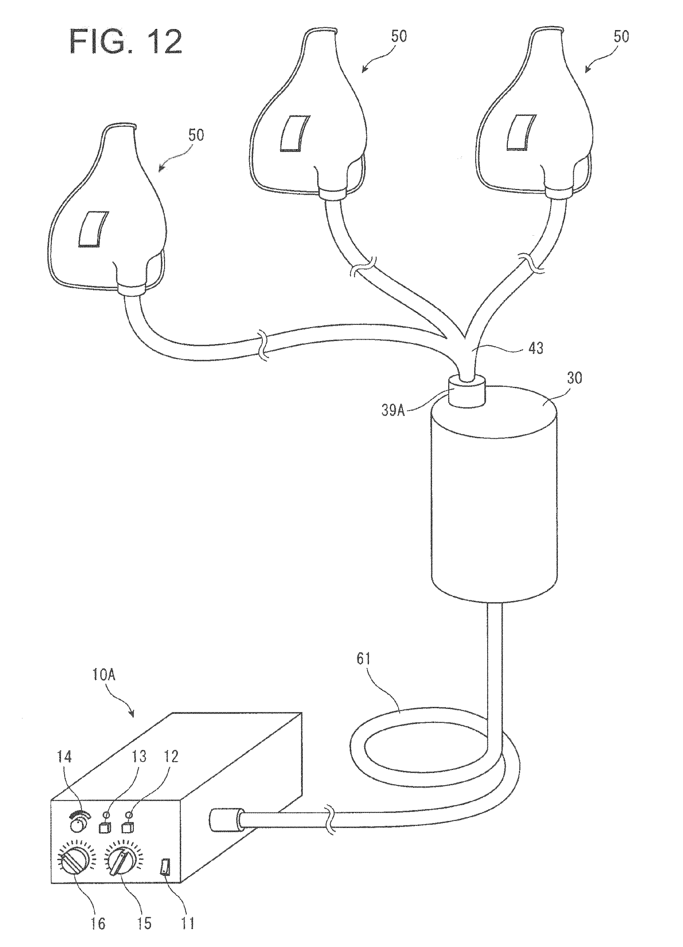

FIG. 12 is the generally schematic view of the gas mist inhaler depending on the third embodiment of this invention. As to the same parts as those of the embodiment shown in FIGs. 1 to 11, the same numerals will be given, and detailed explanation will be omitted.

As showing in FIG. 12, the gas mist inhaler of this embodiment has the gas supply device 10A as the gas supply means, the gas mist generator 30 being the gas mist generating means and plural inhalation members 50 (herein, three inhalation masks are shown).

The present embodiment equips a gas mist supply pipe 43 diverging into plural (herein, three) pipes between the gas mist generator 30 and three inhalation masks 50. It is thereby possible to connect the plurality of inhalation members 50 to one gas supply device 10A and the gas mist generator 30. At this time, the gas supply device 10A adjusts the supplying pressure by the gas supply pressure adjusting part 16 such that a gas inhalation can be performed optimally in each of the plural inhalation members 50.

The present embodiment illustrates the example of using the gas mist supply pipes 43 diverging into the plurality of pipes, and the gas mist discharge part 39 of the gas mist generator 30 may provide plural discharge mouths 39A, each of which is connected with the gas mist pipe 41, or separately provide plural diverged pipes on the way of the ordinary gas mist pipes for supplying the gas mist into the plural inhalation members.

As having above mentioned, according to the gas mist inhaler of the present invention, since the gas introduction part for heightening gas mist supplying pressure is formed integrally with the gas mist generator, the structure is simplified and reduces labors when using, thereby to enable lower manufacturing costs and avoid gas leakage.

The above references have explained the embodiments of the invention, but are not limited thereto, and so far as not deviating from the subject matter of the invention, various kinds of embodiments are, of course, available.

### INDUSTRIAL APPLICABILITY

The present invention relates to a gas mist inhaler for carrying out inhalation of a gas mist into a living body, in which the gas mist is prepared by pulverizing and dissolving oxygen, carbon dioxide and liquid, or a mixed gas of oxygen and carbon dioxide and liquid, and has industrial applicability.

### EXPLANATION OF THE REFERENCE NUMERALS AND SIGNS

10: gas supply means
10A: gas supply device
11: power switch
12: oxygen supply ON/OFF switch
13: carbon dioxide supply ON/OFF switch
14: gas mixing ratio setting part
15: OFF timer (gas supply time setting part)
16: gas supply pressure adjusting part
20: case
21, 22: stands
23: hook
30: gas mist generator
30A: dial switch
30B: residual gage
31: connection part
32: diverging part
33: liquid storage
34: nozzle
34A: front end open of the nozzle
35: liquid suction pipe-forming member
35A: liquid suction pipe
35B: front end of the liquid suction pipe-forming member (liquid suction pipe)
36: baffle (collision member)
36A: baffle supporter
37: confluent part
38: gas introduction part
39: gas mist discharge part
39A: gas mist discharge mouth
41: gas mist supply pipe
41A: cornice shaped pipe
42: groove
43: gas mist supply pipe
50: inhalation mask (inhalation member)
50A: mouth mask
50B: nose mask
51, 51A, 51B: connection part
52, 52A, 52B: inhalation port
53, 53A, 53B: opening
61: gas supply pipe

## Claims

1. A gas mist inhaler, comprising:
a gas supply means of supplying oxygen, carbon dioxide, or mixed gas (called as "gas" hereafter) of oxygen and carbon dioxide,
a gas mist generation means of generating a mist (called as "gas mist" hereafter) prepared by pulverizing and dissolving the gas supplied from the gas supply means as well as a liquid stored in an inside thereof and of supplying the mist under a condition of mixing with the gas,
and
an inhalation member connected to the gas mist generation means and having an inhalation port for causing the above mentioned gas mist to be absorbed into a living body,
wherein the above mentioned gas mist generation means is integrally provided with
a connection part to the gas supply means,
a diverging part of branching a gas flow from the connection part,
a liquid storage of storing the liquid,
a nozzle of discharging one-side gas flow branched by the diverging part,
a liquid suction pipe of sending the liquid to a front end of the nozzle,
a collision member of colliding the liquid blown up by the gas flow discharged from the nozzle with the collision member,
a confluent part of joining the generated gas mist with the gas from an upward side,
a gas introduction part of leading the other-side gas flow branched by the diverging part until the confluent part, and
a gas mist discharge part of collecting and discharging the generated gas mist and gas.

2. The gas mist inhaler according to claim 1, wherein, in the gas mist generation means, the at least liquid storage is made replaceable and exchangeable with another liquid storage.

3. The gas mist inhaler according to claim 1 or 2, wherein the gas mist generating means has an air hole for taking in outside air.

4. The gas mist inhaler according to any one of claims 1 to 3, wherein the inhalation member to has a further opening for taking in outside air.

5. The gas mist inhaler according to any one of claims 1 to 4, wherein the gas supply means is a gas bomb of a cartridge system.

6. The gas mist inhaler according to any one of claims 1 to 5, wherein the gas supply means has any one or a plurality of a gas supply time setting part, a gas supply pressure adjusting part and a gas mixing ratio setting part.

7. The gas mist inhaler according to claim 6, wherein the gas mist generating means supplies the gas mist into the plural inhalation members.

8. The gas mist inhaler according to claim 1, wherein the above mentioned liquid is any one or plural combination of water, ionic water, ozone water, physiological salt solution, purified water, or sterilized and purified water.

9. The gas mist inhaler according to claim 1, wherein a size of the mist supplied from the gas mist generating means into the inhalation member is not larger than 10 µm.

10. The gas mist inhaler according to claim 1, wherein the gas mist generating means has a gas mist supply pipe for supplying the gas mist and gas into the inhalation member, and
the gas mist supply pipe is furnished with a filter for removing liquid drops attached to the inside of the pipe.

11. The gas mist inhaler according to claim 1, wherein the gas mist generating means has a gas mist supply pipe for supplying the gas mist and gas into the inhalation member, and
the gas mist supply pipe is composed of a cornice shaped pipe over a whole or at one part of the gas mist supply pipe.

12. The gas mist inhaler according to claim 11, wherein the cornice shaped pipe is formed inside with a groove in an axial direction of the pipe.

13. The gas mist inhaler according to claim 1, wherein the gas mist generating means has a gas mist supply pipe for supplying the gas mist and gas into the inhalation member, and
the gas mist supply pipe is provided with a check valve.

14. The gas mist inhaler according to claim 1, wherein the gas mist generating means is in advance sterilized.
